# EUROPEAN PATENT APPLICATION

(11) **EP 2 264 149 A1**
(43) Date of publication of application: **22.12.2010**
(21) Application number: 10183141.0
(22) Date of filing: 12.11.2006
(51) Int. Cl.: C12N 5/077, A61L 27/24, A61L 27/38

(54) **Method for non-autologous cartilage regeneration**

(30) Priority: 10.11.2005 US 735204 P; 15.06.2006 US 813690 P
(62) Divisional of application: 09010305.2
(71) Applicant: Carticure Ltd., 16000 Nazareth (IL)
(72) Inventor: Maor, Gila, 26360, Kiryat-Motzkin (IL)
(74) Representative: Becker Kurig Straus

(57) **Abstract**

The present invention relates in general to the field of tissue engineering, and in particular to methods for repair and regeneration of diseased and injured bone and cartilage tissue by allotransplantation or xenotransplantation of neonatal mandibular condyle-derived chondrocytes. The composition may comprise mandibular condyle-derived chondrocytes in the form of a chondrocyte film, per se, or in combination with a scaffold.

## Description

### FIELD OF THE INVENTION

The present invention relates in general to the field of tissue engineering, and in particular to methods for repair and regeneration of diseased and injured bone and cartilage tissue by allotransplantation or xenotransplantation of perinatal mandibular condyle-derived chondrocytes.

### BACKGROUND OF THE INVENTION

Tissue engineering may be defined as the art of structurally and functionally reconstructing mammalian tissues (Hunziker, 2002). In general, tissue engineering provides cells per se or a scaffold that serves as a support onto which cells may attach, proliferate, and synthesize tissue in situ, to restore tissue lost due to disease, trauma or the aging process.

Articular joints are a vital component of the musculoskeletal system. Freely moving joints (ankle, elbow, hip, knee, shoulder, and those of the fingers, toes and wrist) are known as diarthrodial or synovial joints and are critical for body movement. Synovial joints have several distinguishing characteristics, including articular cartilage, a hyaline cartilage covering the ends of the opposing bones and a joint cavity, a space that is filled with lubricating synovial fluid. Articular cartilage is structurally organized and consists of specialized cartilage cells termed "chondrocytes" embedded in an intercellular "cartilage matrix", which is rich in proteoglycans, collagen fibrils (primarily type II), other proteins, and water. In animal joints, the synovial fluid, articular cartilage, and the supporting bone form the weight-bearing system of the body. While synovial joints are subject to an enormous range of loading conditions on a daily basis, the cartilage surfaces undergo little wear and tear under normal circumstances. Breakdown of the joint cartilage as a result of disease, senescence or wear leads to arthritis.

Cartilage and bone diseases include highly debilitating diseases such as osteoarthritis, articular cartilage injury, meniscal disorders and joint infections for which no optimal therapies are currently available. Repair of arthritic joints requires orthopedic surgery to regenerate the degenerated cartilage by a prosthesis or by a biological graft.

Certain compositions and methods useful to restore bone and cartilage tissue have been disclosed in the art. In general, cartilage or bone tissue is treated by administering chondrocytes isolated from autogeneic articular or auricular cartilage. Autologous chondrocyte implantation, or ACI, is a procedure wherein cartilage cells are isolated from a subject's knee, cultured as discrete cells or as aggregates, and subsequently implanted into the subject *per se* or in combination with a scaffold or matrix.

The inventor of the present invention has developed a model for the redifferentiation of chondrocytes, which were isolated from 3-day old mouse mandibular condylar tissue (Reiter et al, 2002). US Patent Application Publication No. 2004/0175826, of the inventor of the present invention, teaches a method of generating cultured chondrocytes from mandibular condyle tissue isolated from 3 day old mice. That application further discloses treating a subject by administering syngeneic or allogeneic mandibular condyle-derived chondrocytes to a subject in need thereof. Alternatively, cultured cells from a genetically engineered animal such as a pig were suggested.

Cartilage has been considered an immuno-privileged tissue. Allograft cartilage transplantations have been successfully performed both in experimental models of damaged articular cartilage in animals (Lu, et al, 2005; Moskalewski et al, 2002) and in patients suffering from articular lesions. Moreover, it has been shown that a cartilage capsule may serve as an immunoisolation barrier of transplanted insulin producing-Langerhans cells, utilizing the immunoprivileged properties of the chondrocyte matrix (Pollok, et al., 2001a; Pollok et al, 2001b).

The poor immunogenicity of cartilage is probably due to the fact that cartilage is an avascular tissue that is also devoid of lymph vessels. However, it is currently widely accepted that the cartilage being an immunoprivileged tissue is mainly due to the fact that most of its cellular antigenic sites are hidden within dense extracellular matrix. Elves (1976) stated that cartilage graft is therefore antigenic but only feebly immunogenic, as the matrix proteoglycans protect the cells from the afferent arm of the immune response. This explains why, in fact, exposed cartilage cells do provoke an immune response, for example an autoimmune reaction in rheumatoid arthritis and T cell reaction in ankylosing spondylitis (van Bilsen et al., 2004; Atagunduz, et al., 2005). It also explains why successful allograft procedures of cartilage replacement have been achieved with pieces of cartilage tissue rather than with separated chondrocyte implants (reviewed by Moskalewski, 2002).

Certain compositions for xenotransplantation for human chondral defects are known, but none have proven to be effective. Fuentes-Boquete et al (2004) used porcine chondrocytes to repair human cartilage lesions. Following 8-12 weeks, repair tissue filled near 30-40 percent of the defect. At 8 weeks, the newly synthesized tissue was composed of a fibrous mesh including some cells. However, at 12 weeks it showed a hypercellular hyaline-like region. The repaired tissue showed positive immunostaining for both type I and II collagen, whereby type I collagen is a marker for the presence of fibrocartilage.

Soft tissue xenografts for cartilage repair is disclosed in US Patent No. 6,758,865. That tissue is prepared by treating the graft with a glycosidase, followed by sialylation treatment, in order to prepare substantially non-immunogenic xenografts for soft tissue repair.

US Patent No. 6,645,764 discloses human neocartilage, having multiple layers of cells surrounded by a substantially continuous insoluble glycosaminoglycan and collagen-enriched hyaline extracellular matrix. The neocartilage serves as replacement tissue for diseased or injured cartilage. That invention is exemplified by allogeneic neocartilage, although according to that disclosure the neocartilage may comprise avian or mammalian chondrocytes derived from transgenic animals, which have been genetically engineered to prevent immune-mediated xenograft rejection. The neocartilage composition is a substantially continuous layer of tissue at least two cell layers thick. After 14 days of growth, the neocartilage was between 10 and 15 cell layers thick. The neocartilage can be grown to various size specifications to facilitate implantation.

US Patent Application Publication 2004/0082063 teaches three-dimensional macromass cultures of, *inter alia,* chondrocytes. The tissue-like masses are disclosed to be useful for tissue replacement.

The above references neither teach nor suggest effective xenogeneic cartilage regeneration using cells or a flexible xenogeneic cartilaginous film.

There remains an unmet need for a method of treating cartilage and bone disorders using xenogeneic or allogeneic cells.

### SUMMARY OF THE INVENTION

The present invention provides methods for the treatment of cartilage and bone disorders comprising mandibular condyle-derived chondrocytes (MCDC) isolated from perinatal mammals for the preparation of a xenograft or allograft composition. Surprisingly, both the allograft and xenograft are substantially non-immunogenic and may be administered in the absence of immunosuppressive therapy. The cells are versatile and can be utilized *per se,* in combination with a scaffold or can be prepared as a chondrocyte sheet, also designated hereinbelow as film.

The present inventor discloses for the first time that a flexible chondrocyte film comprising porcine perinatal MCDC repairs large cartilage defects in a xenogeneic goat model. In addition, murine and porcine mandibular condyle-derived chondrocytes (MCDC) isolated from perinatal and neonatal animals do not evoke a host immune response when transplanted into damaged knees of adjuvant induced arthritic (AIA) rats. Specifically, even sixty days after xenotransplantation, mouse and porcine-derived MCDC's replenished the articular lesions of rats with no sign of white blood cell infiltration.

Furthermore, the cells and the chondrocyte film, when transplanted into a cartilage lesion, develop into durable, functional articular hyaline cartilage with no signs of fibrillar matrix organization, the structural feature typifying fibrocartilage. Fibrocartilage has poor mechanical properties and degenerates over time. Importantly, the cells adapt themselves to the original anatomic shape of the articular cartilage and do not hyperproliferate *in vivo,* thereby reducing the risk of a tissue overgrowth in the joint.

Accordingly, in one aspect the present invention provides a flexible chondrocyte film for the treatment of an orthopedic disorder, the chondrocyte film comprising from one to about ten layers of mammalian perinatal mandibular condyle derived chondrocytes (MCDC), wherein the MCDC are surrounded by an insoluble matrix, wherein the insoluble matrix comprises type II collagen. In some embodiments the film comprises about 2 to about 5 layers of MCDC, preferably from about 2 to about 3 cell layers of MCDC. The cartilage film retains its capacity to undergo *de novo* proliferation and differentiation *in vivo.* In various embodiments the chondrocyte film is capable of regenerating hyaline cartilage *in vivo.* In other embodiments the regenerated hyaline cartilage is substantially free of fibrocartilage.

In certain embodiments the matrix further comprises at least one proteoglycan. In preferred embodiments the matrix comprises aggrecan.

In various embodiments the MCDC are isolated from a perinatal donor mammal, wherein the donor of the MCDC are allogeneic or xenogeneic to the recipient subject. In some embodiments the mammal is a neonate and the neonatal age is less than 7 days old.

In some embodiments the neonate is about 3 days old, or preferably about 24 hours old. In other embodiments the mammal is a fetus.

Suitable mammals for the isolation of MCDC include a human and non-human mammal. Suitable mammals include, *inter alia* rat, mouse, rabbit, guinea pig, goat, lamb, sheep, calf, cow, dog and pig. In one preferred embodiment the mammal is a human.

In preferred embodiments the compositions of the present invention are substantially non-immunogenic , namely do not elicit an immune response that is detrimental to the retention of the graft. In some embodiments the composition is administered to a subject in the absence of an immunosuppressive therapy.

In another aspect the present invention provides a method for the treatment of an orthopedic disorder in a subject in need thereof, the method comprising the step of administering to a site requiring chondrocytes in the subject a flexible chondrocyte film, the chondrocyte film comprising from one to about ten layers of mammalian mandibular condyle derived chondrocytes (MCDC), wherein the MCDC are surrounded by an insoluble matrix, wherein the insoluble matrix comprises type II collagen and aggrecan.

In various embodiments the MCDC are isolated from a perinatal donor mammal, wherein the donor of the MCDC are allogeneic or xenogeneic to the recipient subject. In some embodiments the mammal is a neonate and the neonatal age is less than 7 days old.

In some embodiments the neonate is less than about 7 days old. In some embodiments the neonate is less than about 3 days old, or preferably less than about 24 hours old. In other embodiments the mammal is a fetus.

Suitable mammals for the isolation of MCDC include human and non-human mammals. Suitable mammals include, *inter alia* rat, mouse, goat, lamb, calf, dog and pig. In other embodiments the mammal is a human.

In some embodiments the MCDC are allogeneic to the recipient subject. In other embodiments the MCDC are xenogeneic to the recipient subject.

In various embodiments the subject is a human subject and the MCDC are allogeneic. In other embodiments the subject is human and the MCDC are xenogeneic. In some embodiments the xenogeneic MCDC derive from a mammal selected from a dog and a pig.

In preferred embodiments the compositions of the present invention are substantially non-immunogenic. In some embodiments the composition is administered to a subject in the absence of an immunosuppressive therapy.

In some embodiments the orthopedic disorder is a lesion in the cartilage resulting from disease or injury. In some embodiment the disease is a cartilage degenerative disease selected from osteoarthritis and rheumatoid arthritis. In certain embodiments the lesion results from trauma, for example, from a sports injury or accident. In other embodiments the orthopedic disorder is a bone disorder, including for example a bone fracture or lesion. In other embodiments the injury or diseased tissue includes cartilage and subchondral bone.

The chondrocyte film is topically administered to the site of injury or disease, via surgical techniques including open knee surgery and arthroscopy.

In another aspect the present invention provides a method for preparing a flexible chondrocyte film comprising about one to about ten layers of mammalian perinatal MCDC, wherein the chondrocytes are surrounded by an insoluble matrix, the matrix comprising type II collagen and aggrecan, the method comprising the steps of:
a. isolating MCDC from the mandibular condyle of a perinatal mammal;
b. culturing the chondrocytes under conditions to induce attachment of the chondrocytes to a surface and secretion of a type II collagen matrix;
c. culturing the chondrocytes from one up to about ten cell layers thick;
d. separating the film from the surface.

The present invention further provides the use of mammalian perinatal MCDC for the preparation of a flexible chondrocyte film useful in the treatment of an orthopedic disorder. The chondrocyte film comprises about one to about ten layers of MCDC, wherein the chondrocytes are surrounded by an insoluble matrix, the matrix comprising type II collagen.

In certain embodiments the matrix further comprises a proteoglycan. In various preferred embodiments the matrix comprises aggrecan.

The present invention further provides the use of a flexible chondrocyte film comprising about one to about ten layers of mammalian perinatal MCDC, wherein the chondrocytes are surrounded by an insoluble matrix, the matrix comprising type II collagen and aggrecan for treating an orthopedic disorder.

In another aspect the present invention provides a method for the treatment of an orthopedic disorder in a subject in need thereof, the method comprising the step of administering to the subject a composition comprising a therapeutically effective amount of mandibular condyle derived chondrocytes (MCDC) isolated from a perinatal donor mammal, wherein the perinatal donor is allogeneic or xenogeneic to the subject.

In some embodiments the mammal is a neonate and the neonatal age is less than 3 days old. In preferred embodiments the neonate is about 24 hours old. In some embodiments the cells are transplanted *per se.* The composition of the present invention does not require the use of a scaffold but in certain embodiments the cells are transplanted together with a scaffold, for example a biocompatible scaffold.

The present invention provides the use of a therapeutically effective amount of perinatal mandibular condyle derived chondrocytes (MCDC) isolated from an allogeneic or xenogeneic perinatal mammal for the preparation of a composition useful in the treatment of an orthopedic disorder in a subject in need thereof. In some embodiments the composition comprises fetal or neonatal MCDC.

These and other aspects and embodiments of the present invention will be apparent from the figures, detailed description, examples and claims that follow.

### BRIEF DESCRIPTION OF THE FIGURES

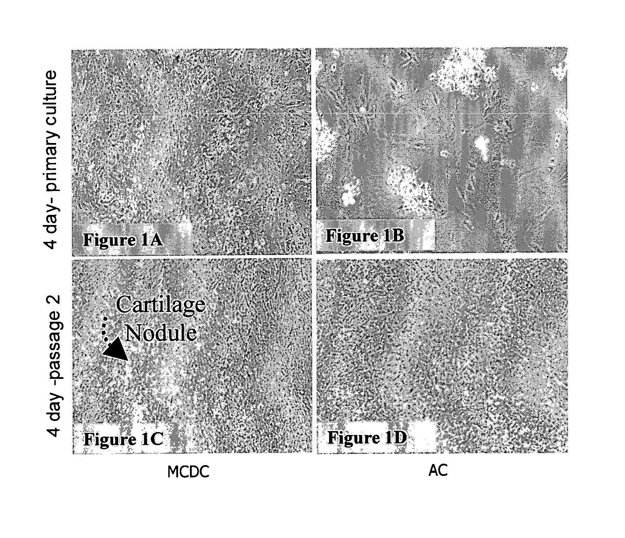

Figures 1A-1D show the difference between mandibular condyle- (MC) and articular cartilage (AC)-derived chondrocytes in primary culture (4 days; 1A and 1B) and in passaged culture (4 days; 1C and 1D). Mandibular-derived primary culture is confluent and contains polygonal cells (1A). The articular-derived culture is very sparse with many non-adherent cells forming refractive-shiny aggregates of cells (1B).

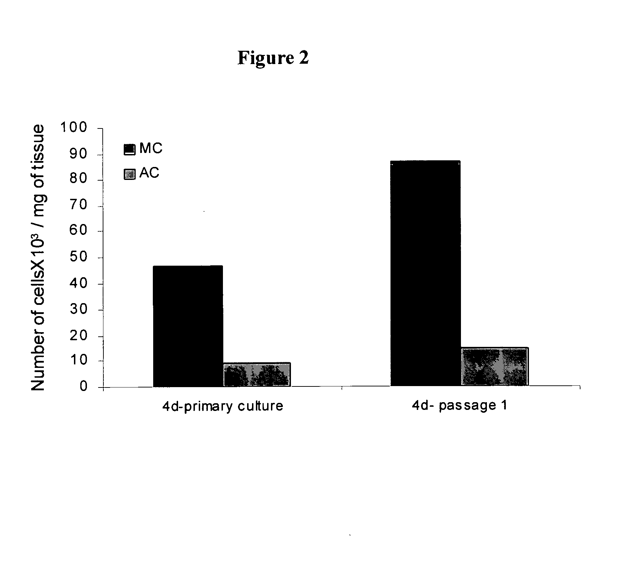

Figure 2 shows that the yield of cartilage cells from the mandibular condyle is higher than that from the articular condyle, in primary culture and following passage of cells.

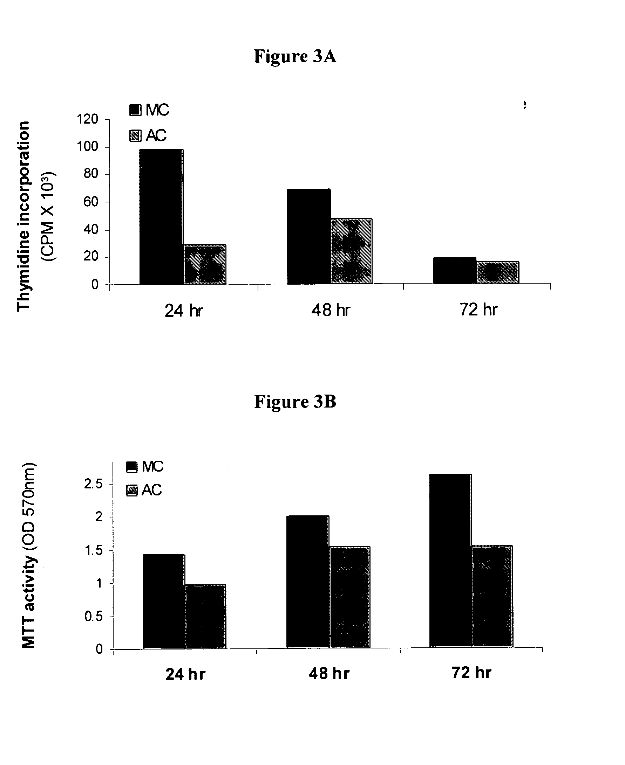

Figures 3A and 3B show proliferation levels of MC- and AC-derived chondrocytes. Figure 3A shows proliferation as a measure of thymidine incorporation into DNA in the chondrocytes from the two sources. The lower proliferation activity is reflected eventually in fewer AC-derived viable cells compared to the MC-derived chondrocytes as apparent from the lower MTT activity (3B).

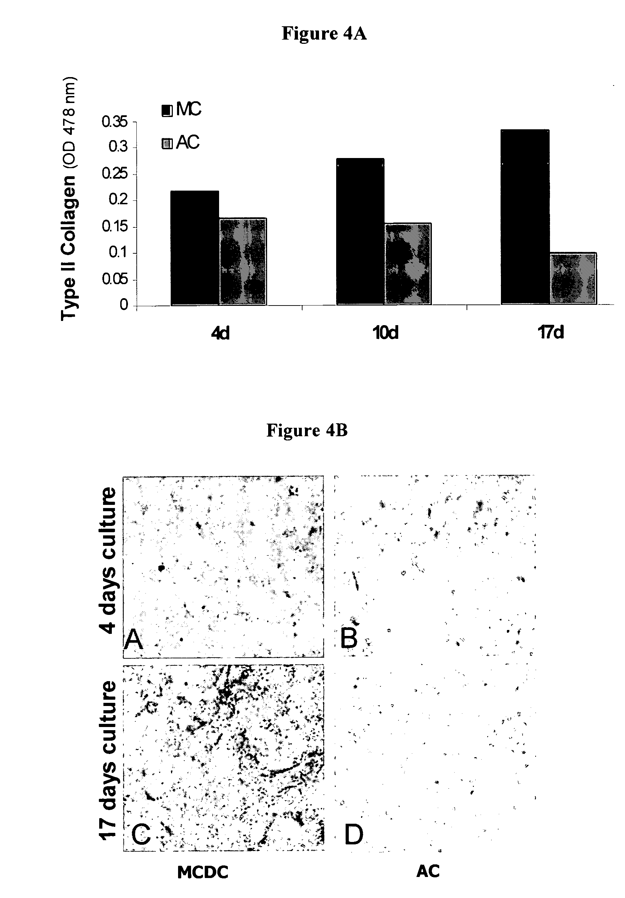

Figures 4A and 4B shows that mandibular condyle-derived chondrocytes differentiate into hyaline cartilage, as measured by type II collagen expression that increases with culture duration. In contrast, AC derived cells produce less type II collagen that decreases with culture duration.

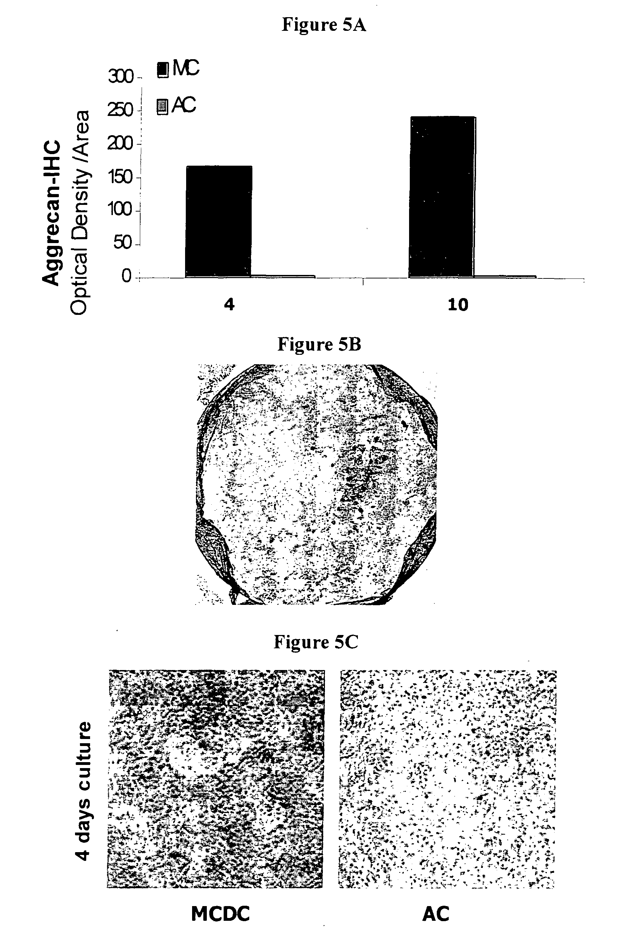

Figures 5A - 5B show the presence of aggrecan in MC -derived chondrocytes. Immunohistochemical analysis of 4 and 10 day old cultures (5A), reveal markedly higher positive aggrecan activity in MC-derived chondrocytes as compared to the AC-derived chondrocytes. Immunohistochemical staining shows that after 4 days in culture the MCDC (left panel) express aggrecan while the AC derived chondrocytes (right panel) show weak expression (5C) Figure 5B shows sulphated proteoglycan staining in cartilage film.

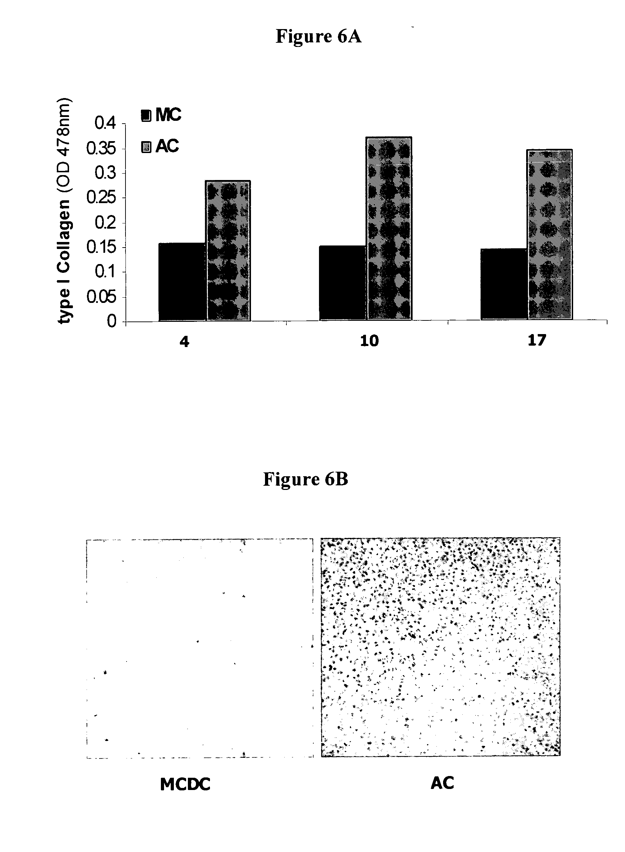

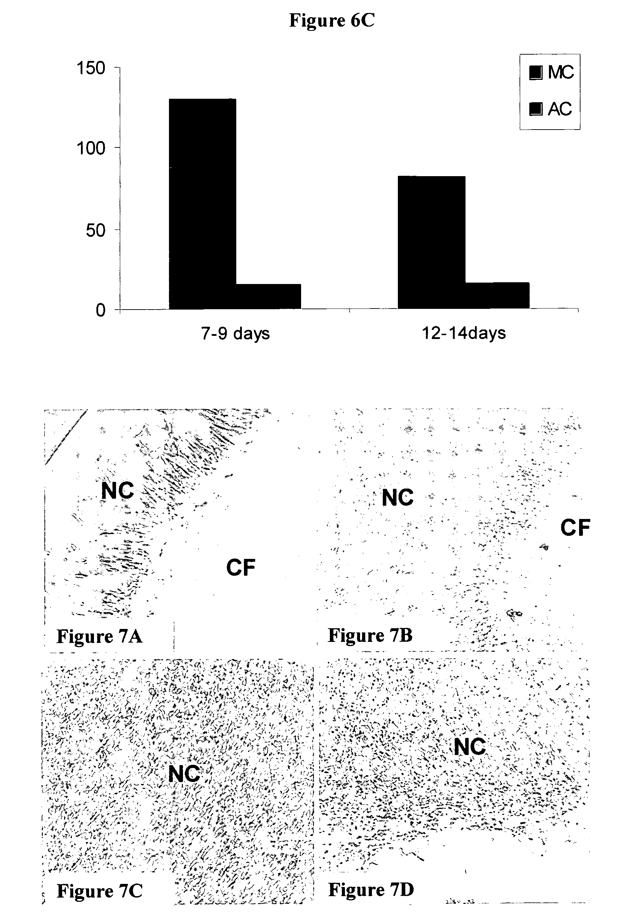

Figures 6A and 6B show that the expression of type I collagen determined by immunohistochemical staining in cultured AC is higher than that in MC- derived chondrocytes indicating a development of fibrocartilage rather than hyaline cartilage in the AC cultures.
Figures 7A-7D show microscopic views of cartilaginous film formed by MC-derived chondrocytes. Four days post re-plating (7A) new cells (NC) start to sprout out from the 'old' cartilage film (CF). After 8 days these new chondrocytes start to gain polygonal shape (7B). Two weeks post re-plating a new typical chondrocyte culture develops (7C) occupying most of the new culture well. However the new culture does not feature cellular over-growth, and is restrained by a clear border of cells (figure 7D, arrow), indicating that the secondary new culture (NC) preserves features of a primary culture and does not proliferate uncontrollably.

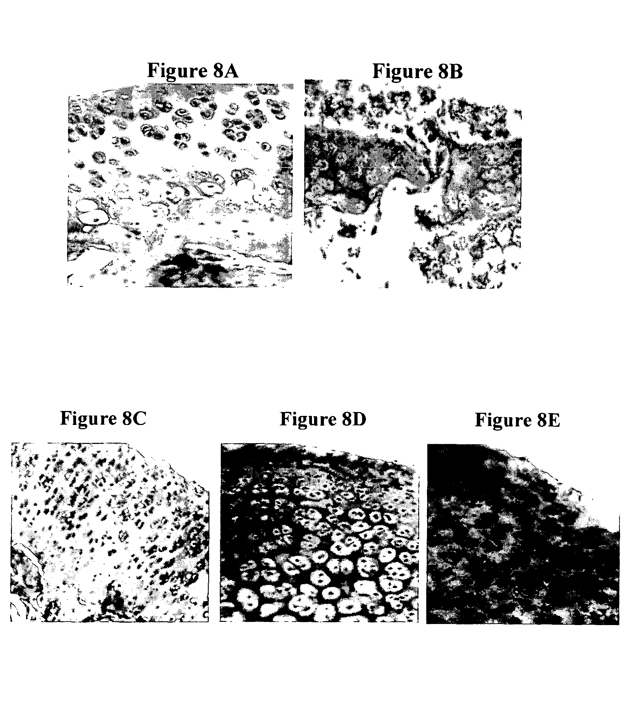

Figures 8A-8E show histochemical cross-sections of rat articular cartilage following sixty days after transplantation. Control naïve animal (8A), an AIA induced rat (8B), allogeneic transplantation of rat MCDC cells transplanted into rat (8C), xenogeneic transplantation of mouse MCDC cells transplanted into rat (8D) and xenogeneic transplantation of porcine MCDC cells transplanted into rat (8E). In both allogenic and xenogenic transplantations, new, and histologically well organized hyaline cartilage covers the apical articular surface of the damaged cartilage, with no signs of immune rejection as measured by the absence of WBC infiltration.

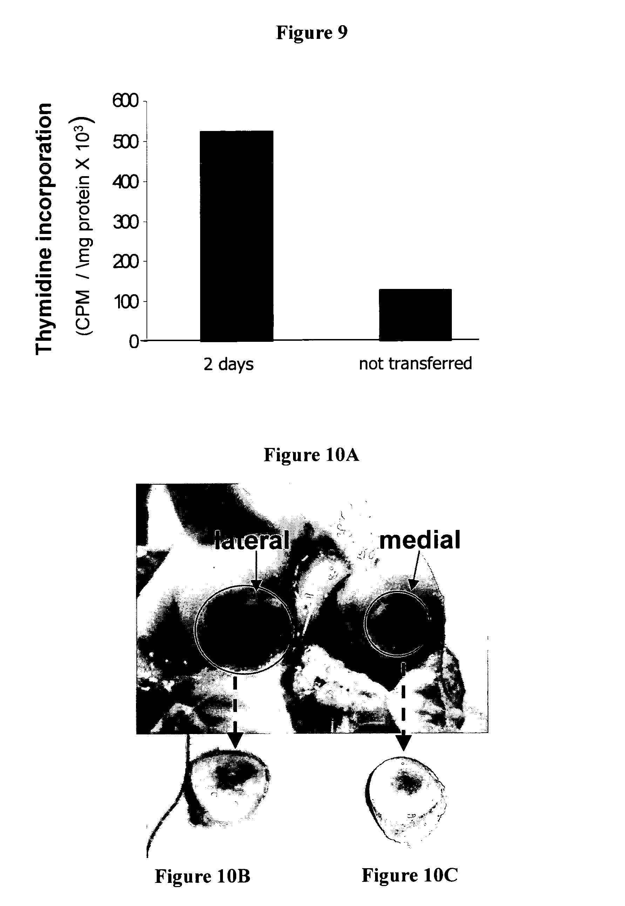

Figure 9 shows that MCDC in chondrocyte film regain proliferative capacity when replated.
Figures 10A-10C show the surface of the cartilage lesions in a goat's knee The lateral condyle (10B) was left untreated while the medial condyle (10C) was treated. The photograph was taken 14 weeks after 10 mm lesions were created and a porcine derived cartilage film comprising MCDC implanted.

### DETAILED DESCRIPTION OF THE INVENTION

Though numerous methods for the treatment of skeletal disorders utilizing chondrocytes are known in the art, none are completely effective for the repair of and or restoration of fully functional articular tissue. Previous studies have shown that allogenic and or xenogenic transplants and implants used for the regeneration of cartilage are associated with a number of disadvantages including tissue overgrowth, formation of fibrous cartilage and or the need for immunosuppressive therapy or agents. Neocartilage implants, which have been used to substitute lost cartilage do not provide an adequate solution for a weight bearing tissue.

The present invention provides methods of using allogeneic or xenogeneic perinatal mandibular condyle derived chondrocytes for cartilage and bone regeneration and repair. The MCDC have a number of characteristics, which render them advantageous in clinical applications. Among the advantageous properties of the matrices of the invention:
a. guaranteed homogenous population of hyaline cartilage producing cells ;
b. simple and rapid culturing conditions;
c. absence of hyperproliferation and associated growth of fibrocartilage;
d. easily isolated from perinatal or postnatal animal mandibles;
e. xenogeneic source thereby obviating the need for multiple surgical procedures normally required for isolation of autogeneic cells;
f. qualitatively reproducible product, which is not dependent on the age and health conditions of the donor (as in autologous chondrocyte transplantation (ACT) approach);
g. immediate supply of unlimited amounts of cells;
h. obtained from animals grown in controlled environment thereby obviating the health risks attendant with xenogeneic cell transplant and overcomes the lack of suitable allogeneic source material.

### Definitions

For convenience and clarity certain terms employed in the specification, examples and claims are described herein.

The term "cartilage" as used herein, refers to a specialized type of connective tissue that contains chondrocytes embedded in an extracellular matrix. Several types of cartilage have been identified which differ in their histological and mechano/physical properties and include, hyaline cartilage, fibrous cartilage, and elastic cartilage. Articular cartilage that covers the apical regions of bones at the joints is composed of hyaline cartilage. The biochemical composition of cartilage differs according to type but characteristically comprises fibers: collagens and/or elastic fibers, ground-amorphous substance comprised mainly of glycosaminoglycans proteoglycans, other proteins and water. The term "chondrocytes" as used herein, refers to cells, which are capable of producing components of cartilage tissue.

Hyaline cartilage, the most abundant form of cartilage, is glass smooth, glistening and bluish white in appearance, although older or diseased tissue tends to lose this appearance. The most common hyaline cartilage, and the most studied, is the articular cartilage, which covers the articulating surfaces of bones within synovial joints. Articular cartilage is characterized by specialized cartilage cells termed "chondrocytes" embedded in a "cartilage matrix", which is rich in cartilage specific highly sulfated proteoglycans-denoted aggrecans, type II collagen fibrils along with other minor types, e.g., types IX and XI, other proteins, and water. While cartilage tissue is neither innervated nor penetrated by the vascular or lymphatic systems, in the mature joint of adults, the underlying subchondral bone tissue is innervated and vascularized. Beneath this bone plate, the bone tissue forms trabeculae, containing the marrow. In immature joints, articular cartilage is underlined by only primary bone trabeculae. A portion of the meniscal tissue in the knee joint (referred to as the "interarticular" cartilage) consists mainly of fibrous cartilage.

"Neocartilage" refers to cartilage, which is produced *ex vivo,* and can be implanted *per se* to replace cartilage.

Fibrous cartilage is characterized by the presence of type I cartilage. The presence of type I collagen in repaired cartilage is believed to be unfavorable for tissue repair (Briggs, et al., 2003). The term "substantially free of fibrous cartilage" refers to regenerated cartilage tissue that has less than about 20% fibrous cartilage, preferably less than about 10%, preferably less than about 5% fibrous cartilage as measured by the presence of type I collagen.

It is generally believed that because mature articular cartilage lacks cartilage producing stem cells and vasculature, damaged cartilage tissue does not receive sufficient or proper stimuli to elicit a repair response.

"Cartilage film" and "chondrocyte film" are used herein interchangeably and refer to a flexible matrix comprising chondrocytes embedded within an insoluble matrix, the matrix being about one to about ten cell layers thick. Thee insoluble matrix is secreted by the chondrocytes.

The term "flexible" refers to the capability of being folded, shaped or bent while maintaining integrity. In particular, a flexible film is a pliable film. A flexible chondrocyte film can be molded or bent to fit the contours of the tissue in need of regeneration.

The term "mandibular condyle derived chondrocytes" or "MCDC" refers to chondrocytes isolated from the mandibular condyle of a neonate. The mandibular condyle refers to the growth center in the lower jawbone, where the jaw articulates with the temporal bone of the skull in a vertebrate. The mandibular condyle is a unique source of cartilage since it serves two distinct developmental objectives: during the early postnatal period it serves as the growth center of the mandible following normal cascade of endochondral ossification, and upon growth cessation it turns into articular cartilage. During the early-growing phase of the mandibular condyle, it exhibits all histological and biochemical features typifying hyaline cartilage. The MCDC cells are able to develop into fully functional hyaline cartilage *in vitro* and *in situ* and express the molecular markers associated therewith; functionally and structurally repair or replace damaged or diseased cartilage tissue; exhibit a high proliferation rate *in vitro* yet do not over proliferate *in vivo,* thus obviating the risk of tissue repair with fibrocartilage; can be transferred from one substratum to another while renewing their proliferative and chondrogenic features, thus producing new hyaline cartilage in the new substratum.

The terms "repair" and "regeneration" refer to the proliferation, growth, development and or restoration of tissue *in situ* that has been damaged by trauma, disease or injury.

The term "non-autologous" refers to tissue or cells which originate from a donor other than the recipient. Non-autologous can refer to for example allogeneic or xenogeneic. The terms "autogeneic" and "autologous" as in autogeneic graft, autologous chondrocytes etc, refers to a graft, cartilage, etc., in which the donor and recipient are the same individual. Likewise, "allogeneic" refers to a donor and a recipient of the same species; "syngeneic" refers to a donor and recipient with identical genetic make-up (e.g. identical twins or autogeneic) and "xenogeneic" refers to donor and recipient of different species.

The term "substantially non-immunogenic" or "substantially non-antigenic" means that the graft, implant or transplant does not elicit an immune response that is detrimental to the retention of the graft.

A "subject" refers to a recipient or host of the composition of the present invention. In some embodiments the subject is a human subject.

The term "transplantation" or "implantation" refers to the insertion of the film or the injection, infusion or insertion of the composition of the invention into a subject, whereby the composition serves to replace, fully or partially, tissue that has been damaged, diseased or removed. Xenotransplantation refers to the transplantation of cells, tissues or organs from one species to another such as from mice to rats or pigs to humans. Such cells, tissues or organs are called xenografts (xenotransplants). A graft refers to transplanted or implanted cells or tissue. A graft as used herein refers to transplanted chondrocyte film or transplanted MCDC.

The term "perinatal" as used herein pertains to a period from before birth to shortly after birth. In a human, perinatal refers to the period beginning with completion of the twentieth week of gestation and ending about 28 days after birth.

The term "neonatal" as used herein refers to a period beginning at birth and ending at about 28 days after birth. The term "fetal" refers the period beginning at about 20 weeks post gestational until birth.

The term "biocompatible" as used herein refers to a material which has low toxicity, acceptable foreign body reactions in the living body, and affinity with living tissues. The term "cell-bearing" as used herein refers to the capacity of a material to support cells.

The term "scaffold" refers to a solid or semi-solid material, which can be used as a delivery vehicle for cells and bioactive agents. Biocompatible refers to low toxicity, clinically acceptable levels of foreign body reactions in the living body, and affinity with living tissues.

A "proteoglycan" refers to a special class of proteins that are heavily glycosylated. A proteoglycan is made up of a core protein with numerous covalently attached high sulphated glycosaminoglycan chain(s). Non-limiting example of extracellular matrix proteoglycans include aggrecan and certain collagens, such as collagen IX. The presence of aggrecan in a chondrocyte culture is a marker of articular hyaline cartilage.

Type I collagen is generally a component of fibroblast forming tissue, scar tissue, tendons and the organic part of bone. The expression of type I collagen is unfavorable to the development of articular hyaline cartilage, while the expression of type II collagen is favorable.

A "glycosaminoglycan" or "GAG" as used herein refers to a long unbranched polysaccharide molecules found on the cell surface or within the extracellular matrix. Non-limiting examples of glycosaminoglycan include heparin, chondroitin sulfate, dextran sulfate, dermatan sulfate, heparan sulfate, keratan sulfate, hyaluronic acid, hexuronyl hexosaminoglycan sulfate, and inositol hexasulfate.

### Xenogeneic and Allogeneic Cell Therapy

Xenografts and allografts offer tremendous advantage over autologous transplant. Use of xenogeneic cultures, if immunologically neutral, allows maintenance of a small number of cell cultures for virtually all recipient subjects. This reduces patient trauma from tissue harvest, reduces the expense of generating primary cultures, reduces the waiting time required for a surgical procedure and obviates the risk that the autologous chondrocyte culture will not, for some reason, proliferate.

The human use of xenogeneic and allogeneic cells is generally associated with difficult obstacles, including
management of the risks of introducing new infectious diseases into the general population through adaptation in an immunosuppressed host; and
maintaining the long term survival and function of the cells;

These risks are minimized by careful choice of donor mammals, reproducible manufacturing process, accurate pre-clinical and clinical testing and monitoring as well as a risk management program with regard to infectious agents.

The above risks are further minimized with regard to the MC derived chondrocytes of the present invention. The MCDC are isolated from xenogeneic or allogeneic perinatal mammals and shown to be substantially non-immunogenic and non-antigenic, thereby obviating the need for high doses of immunosuppressive drugs. Without wishing to be bound to theory, these features result from the rapid differentiation rate and accompanying high levels of extracellular matrix, which envelops the cells.

As mentioned hereinabove, the cultured cells of the present invention can be administered in-vivo, and in particular *in situ,* for the repair and or replacement of lost or diseased skeletal tissue. Skeletal tissue refers to hard tissues such as bone and subchondral bone and to articular or meniscal cartilages.

The methods of the present invention are suitable for human and veterinary applications. According to some embodiments the recipient of the MCDC is a human or non-human mammal selected from horse, dog, goat or sheep. In certain embodiments the recipient is a human subject.

The method of treatment comprises administering a therapeutically effective dose of allogeneic or xenogeneic MCDC to a subject in need thereof. As used herein, the phrase "therapeutically effective dose" refers to an amount sufficient to effect a beneficial or desired clinical result, wherein the clinical result is repair or regeneration of cartilage or bone tissue. Preferably, cartilage tissue is repaired. As used herein, the term "treating" refers to alleviating, attenuating, palliating or eliminating the symptoms of a disease, slowing, reversing or arresting the progression of the disease, or curing the disease. As used herein, the, term "disease" refers to any medical disease, disorder, condition, or syndrome, or to any undesired and/or abnormal physiological, morphological, and/or physical state or condition. In some embodiments the disease is a chondral disease, in some embodiments the disease is an osteochondral disease. In particular, the present invention provides a method for treating orthopedic defects *inter alia* articular cartilage lesions arising from trauma such as an accident or sports injury or disease such as osteoarthritis. The method is preferably applied to treat the disease in a mammalian subject, preferably a human subject.

Osteoarthritis (OA) usually affects only one or two major joints, and often affects the knee. The etiology of knee osteoarthritis is not known; it is thought to be simply a process of "wear and tear". Some conditions may predispose the knee to osteoarthritis, for example, a previous fracture that involved the joint, or by lesser injuries that may have torn ligaments or menisci. Abnormal development of the bones, such as bow legs, may cause the knee to wear out sooner than normal. In osteoarthritis of the knee the cartilage cushion is either thinner than normal (leaving bare spots on the bone), or completely absent resulting in bare bones grinding against each other, and often resulting in damage to the subchondral bone. In addition to mechanical pain, fragments of cartilage floating in the joint may cause inflammation in the joint lining. X-rays show the "joint space" to be narrowed and irregular in outline.

The etiology of rheumatoid arthritis (RA) is not known. RA is a systemic, autoimmune disease characterized by a chronic, erosive inflammation of painful and debilitating joints, with progressive degradation of cartilage and bone accompanied by proliferation of the synovium.

Osteonecrosis, or spontaneous osteonecrosis of the knee (SPONK), is a rare condition, which may cause knee pain.

In some embodiments the compositions of the present invention are useful in the repair of bone defects including breaks and gaps.

In one preferred embodiment the present invention provides a chondrocyte film comprising from one to about 10 layers of MCDC, wherein the chondrocytes are surrounded by an insoluble matrix, wherein the insoluble matrix comprises type II collagen and aggrecan. In some embodiments the film comprises about 2 to about 5 layers of MCDC, preferably from about 2 to about 3 cell layers. In some embodiments the cartilage film can undergo proliferation and differentiation in *situ.* The chondrocyte film can be implanted into a host, for example to repair a cartilage defect in the knee. The film can be implanted during open knee surgery or in the less invasive surgical procedure known as arthroscopy. In some applications the chondrocyte film may need to be affixed to its target tissue, for example, by suturing or application of surgical glue.

The chondrocyte film is allogeneic or xenogeneic to the recipient.

In another embodiment allogeneic or xenogeneic MCDC are transplanted to a subject.

### Methods and Therapeutic Compositions

The therapeutic composition according to the present method is useful for inducing regeneration or repair of cartilage and bone lesions. In addition to the mandibular condyle-derived chondrocytes (MCDC) the composition can also include one or more pharmaceutically acceptable excipients. As used herein, a "pharmaceutically acceptable excipient" refers to any substance suitable for maintaining and delivering the cells of the composition to an in vivo site (i.e., a cartilage lesion).

Preferred pharmaceutically acceptable excipients are capable of maintaining the viability of the cells. Examples of pharmaceutically acceptable excipients include, but are not limited to water, phosphate buffered saline, Ringer's solution, dextrose solution, serum-containing solutions, Hank's solution, other aqueous physiologically balanced solutions, and the like. Aqueous carriers can contain suitable additives required to approximate the physiological conditions of the recipient, for example, by enhancing stability and isotonicity. Suitable additives include, for example, sodium acetate, sodium chloride, sodium lactate, potassium chloride, calcium chloride, and other substances used to produce phosphate buffer, Tris buffer, and bicarbonate buffer. Additives can also include preservatives.

The MCDC can be administered as isolated, cultured cells to a subject *in situ,* i.e. directly to the lesion. Alternatively the cells may be associated with a biocompatible scaffold. Suitable scaffolds include those made of synthetic or natural materials. Natural materials include cross-linked or non-crosslinked hyaluronic acid, collagen, chitosan, alginate, fibrin, peptide hydrogels, demineralized bone and the like. Synthetic materials include polyethylene glycols, poly(DL-lactic-co-glycolic acid) (PLGA), polyurethane foam, and the like.

The following examples are presented in order to more fully illustrate some embodiments of the invention. They should, in no way be construed, however, as limiting the broad scope of the invention. One skilled in the art can readily devise many variations and modifications of the principles disclosed herein without departing from the scope of the invention

### EXAMPLES

### Example 1: Tissue culture system

### Material and Methods

Neonatal piglet (24 hr old, male or female, ∼1.6 kg weight) was deeply anesthetized with 5 ml Iustil (200mg/ml sodium pentobarbiton, injected into the heart). Mandibular condyles were aseptically dissected, freed of any soft tissue and subjected to successive collagenase digestion (37°C; 0.1% type II collagenase, cat No. C-6885, Sigma Co., St. Louis, MO, USA). Following the 1^{st} digestion (25 min), which mainly separates adjacent soft tissues, the next 4 successive digestions (45 min each) yielded homogenous population of chondrocytes. These mandibular condyle derived chondrocytes are denoted MC. For the sake of comparison, chondrocytes were also separated from the femoral distal condyles and are referred to herein AC.

Dulbecco's Modified Eagle's Medium (DMEM) (cat No. 010551, Biological Industries, Bet Ha'Emek, Israel) supplemented with 1 mM sodium pyruvate, 10% fetal calf serum (FCS), and 1% penicillin/streptomycin (cat No. 030421, 030201, 030311, respectively, Biological Industries, Bet Ha'Emek, Israel), 100 µg/ml ascorbic acid and 10 mM β-glycerophosphate (cat. No. A-4544, C-6251, respectively, Sigma Co., St. Louis, MO, USA). Cells are plated at a concentration of 5 × 10⁵ cells/ml in 35 mm Dia culture dishes at 37°C, 5% CO₂ and maximal humidity. Medium was changed every 48 hr. Every 3-4 days the cells were split. For the present experiments, cells from the second passage (P2) were used.

### Chondrocyte Proliferation

*MTT assay*: Cell viability was measured using the 3-(4,5-dimethyl-thiazol-2-yl)-2,5-diphenyl-tetrazolium bromide (MTT, cat No. M2128, Sigma , St. Louis, MO, USA) assay. The assay is based on the capability of mitochondrial dehydrogenase in intact cells to oxidize MTT into the blue/brownish insoluble formazan.

Cells were incubated in the presence of 1.25mg/ml MTT for 2 hours at 37°C. Developed color is extracted with the addition 60% v/v dissolving buffer (20% SDS in 50% dimethylformamide pH 4.7) and read at 570nm.

*Incorporation of tritiated thymidine into DNA:* Chondrocytes cultured in 24-well plates, were incubated with 1µCi [³H]-thymidine/ml medium (Amersham, code TRA120, stock 1mCi/ml) for 3 hours at 37°C in the presence of serum-free medium. Excess free radioactivity was washed out with PBS (X2), methanol and ice cold 10% tri-chloroacetic acid (TCA) (X3). Cells were solubilized with 200µl 0.3M NaOH for 15 min and neutralized with HCl. Radioactivity was counted in a β-counter.

### Chondrocyte differentiation

*I. Alcian blue (AB): cartilage proteoglycan staining:* Staining with AB was performed on chondrocytes cultured on cover slips. Following pretreatment with 3% acetic acid for 3 min, chondrocytes were stained with 1% alcian blue at pH 2.5 for 30 min and thoroughly rinsed with tap water. Quantification of AB staining was performed by extracting alcian blue staining with 6M guanidine hydrochloride overnight. Results were read at 630 nm wavelength.
II: Incorporation of ³⁵S-sulfate into glycosaminoglycans (GAG)
   *Labeling and extraction:* Chondrocytes were incubated with 20 µCi/ml of [³⁵S]-Na₂SO₄ for 6 hr at 37°C or 10 µCi/ml of [³⁵S]-Na₂SO₄ for 16 hr at 37°C. At the end of radiolabeling, cells were washed twice with PBS (wash fluid is kept and combined with further radioactivity extracts), the medium and cell layer were digested separately with papain (250 µg/ml) for 2 h at 60°C in the presence of 100µg of chondroitin sulphate-A (cat. No. C9819, Sigma), which serves as a carrier. Chondrocytes were then extracted in 4 M guanidine-HCl buffer / 50 mM sodium acetate buffered at pH 7.2, in the presence of protease inhibitors.

*Precipitation of GAG:* 250µl of homogenates were supplemented with 250µl of cold 3% cetylpyridinium chloride (CPC) (cat. No. C0732, Sigma) and incubated at 4°C overnight.

The precipitate was collected by centrifugation and washed with 1ml of 1% CPC, this step was repeated twice. The final precipitate was dissolved in formic acid and radioactivity was counted.

### Immunoblotting

Cell lysate was prepared in ice cold RIPA buffer supplemented with protease inhibitors: 10 µg/ml PMSF, 5µg/ml Aprotonin, 5µg/ml Trypsin inhibitor and 10 µg/ml Benzamidine. Lysates proteins were separated in reducing sodium dodecyl sulfate-polyacrylamide-gel electrophoresis (SDS-PAGE) and electrotransferred to nitrocellulose membrane. Blots were incubated with one of the following: mouse anti-collagen type II (cat. No. MAB 887 Chemicon International, Inc, Temecula, CA, USA), mouse anti-Proliferating Cell Nuclear Antigen (PCNA) (cat No. MO879, Dako, Glostrup, Denmark), or mouse anti-actin (cat. No. MAB 1501, Chemicon International Inc, Temecula, CA) and detected with either goat anti mouse-HRP (cat No. 115-035-062, Jackson ImmunoResearch Inc., West Grove, PA, USA) or goat anti rabbit-HRP conjugates (cat No. 12-348, Upstate Biotechnology, Lake Placid, NY, USA), and revealed by Western Blot Chemiluminescence Reagent Plus (cat no. NEL104, NEN^{™} Life Science Products, Boston, MA, USA).

### In situ Hybridization (ISH)

*Probe labeling:* Digoxygenin (DIG)-labeled anti sense RNA probes were prepared in a two steps polymerase chain reaction (PCR) method: amplification of specific gene insert, amplification of the gene insert conjugated to promoters at each side. The following probes were used for ISH:
**type II collagen** cloned in pBluescript SK⁺ amp⁺(361 bp),
forward 5'- TCT CCT GCC TCC T -3' (SEQ ID NO:1) and
reverse 5'- ACC ATC TCT GCC ACG G -3' (SEQ ID NO:2);
**aggrecan**
forward 5'- GTC CTC T CCA GT (SEQ ID NO:3) and
reverse 5'- ATT GCT TCT CCA GA (SEQ ID NO:4)).

The second primer pair has T3 and T7 promoter sequences at the ends. Antisense mRNA was labeled using 1 µg of gel-purified PCR product (QIAquick® gel extraction kit, Qiagen) and DIG RNA labeling kit (Boehringer Mannheim, Germany), following the manufacturer's instructions.

*Hybridization:* MCDC cells cultured on cover slips were pre-treated with cold methanol to increase membrane penetration followed by quenching endogenous peroxidase with 3% H₂O₂ in methanol. Cells were then treated with12.5 µg/ml proteinase K for 15 min, (stopped with 2 mg/ml glycine), and acetylated with 0.5% acetic anhydride in 0.1 M Tris at pH 8.0; sections are post-fixed with 4% paraformaldehyde/phosphate buffered saline (PBS). Prehybridization for 10' in 2 x standard saline citrate (SSC) is followed by one hr in hybridization buffer: 50% formamide 0.5 mg/ml salmon sperm DNA, 4xSSC, 1x Denhardt's, 200 U/ml heparin, 5% dextran sulfate and 0.01% sodium dodecyl sulfate (SDS). Hybridization was performed overnight (18hr) at 42°C and maximal humidity with 5ng/µl digoxigenin labeled antisense RNA probe (see above). For all probes used, digoxigenin labeled sense RNA probes served as negative controls. At the end of the incubation period, slides were rinsed with SSC under increasing stringency and then with 0.1M Tris 0.15 M NaCl at pH 7.5. Hybrids are detected using anti-digoxygenin antibodies conjugated to peroxidase (Boehringer Mannheim, Germany) and AEC as a substrate; cells were counterstained with hematoxylin.

### Biomechanical analysis

For biomechanical evaluations of the newly formed cartilage, necropsies of osteochondral (articular cartilage and its subchondral bone) are harvested from the treated lesions, the untreated lesions and from parallel healthy tissues in the counter (intact) knee. Specimens are immersed in Phosphate-Buffered Saline (PBS; 2.67 mM KCl, 1.47 mM KH₂PO₄, 138 mM NaCl, 8.1 mM Na₂HPO4·7H₂O at a pH 7.2) and protease inhibitors (1 mM phenylmethanesulfonyl fluoride, 2 mM disodium ethylenediamine tetraacetate, 5 mM benzamidine-HCl, and 10 mM N-ethylmaleimide). Tissues are assayed within 5 hrs of their harvesting.

Stiffness of the tissue is assessed by indentation test using a universal testing apparatus 4502'-Instron-Bioplus™, and a 0.3mm plane ended indenter. The loading protocol consists of application of a "tare displacement" (for 30 s) and then four "test displacements" of 3 seconds (s) duration each. For each tare displacement-load at 0 s and 3 s is noted. Tare displacement is 25 micrometer, test displacements are 50,100, 200, and 300 Micrometers. Indentation Stiffness is computer calculated as the slope of load-displacement data, in units of N/mm (with no need to regard indenter diameter).

### Human-derived MCDC cells.

Chondrocyte culture based on human derived mandibular condyle (human MCDC) is supported by Helsinki approval.

Mandibular condyles are ascetically removed from 20-week-old fetuses of deliberate abortions, explants are immediately transferred into cold Hank's buffer. Cartilaginous zone is removed by cutting at the mineralization front of the condyle using dissecting microscope. Cartilage tissue is subjected to gradually enzymatic digestions (0.1 % collagenase type II), as described for porcine-derived MCDC. Separated chondrocytes are cultured under the same conditions as porcine MCDC.

### Results

### I. Mandibular condyle-derived chondrocytes, but not articular cartilage-derived

### chondrocytes, develop into typical hyaline cartilage

The chondrocyte yield from mandibular condyle is higher than that from knee articular cartilage. Morphological appearance of the cultures was followed by phase microscopy. Results presented in figure 1 clearly show that the mandibular-derived primary culture is much more crowded and contains polygonal cells. The articular-derived culture, however, is very sparse. Most of the cells do not adhere to the plate. The non-adherent cells form refractive-shiny aggregates of cells, while the adherent cells are elongated resembling fibroblast like cells. Four days following the first culture split the mandibular cells are already organized in typical culture nodule, while most the articular cells remain elongated.

The differences in the cellular yield between the two cartilage sources are exemplified in the number of cells separated from one mg wet weight of cartilage tissue. Results presented in figure 2 show that the initial yield of cartilage cells from the mandibular condyle is about 5.7 times higher than that from the articular condyle. Four days after the 1^{st} split the difference between the MC-derived chondrocyte population and that of the AC-derived culture is even greater.

The difference in cell population between mandibular- and AC-derived chondrocytes represents not only a yield gap between the two sources, but also different proliferation rates. Figure 3A shows the levels of thymidine incorporation into DNA in the chondrocytes of the two sources. Normally proliferation rate declines in the developing culture as the cells undergo differentiation. Yet, at each time interval tested: 24, 48 and 72 hr, the levels of thymidine incorporation into the AC-derived chondrocytes was lower (by 70%, 30% and 13% respectively) than that of the MC-derived chondrocytes. The lower proliferation activity is reflected eventually in fewer AC-derived viable cells compared to the MC ones as apparent from the lower MTT activity (figure 3B).

### II. Mandibular condyle-derived chondrocytes differentiate into hyaline cartilage

Normal joint surfacing cartilage is hyaline cartilage, which is characterized by type II collagen and aggrecan-rich matrix. The expression of type II collagen determined using immunohistochemical (IHC) analyses (figure 4B) clearly show intensive positive staining at both 4 (panels A and B) and 17 days (panels C and D) MC culture and a very faint (mainly background) positive staining in the AC-derived chondrocytes. Densitometry of the type II collagen staining (figure 4A) reveals a 24%, 44% and 73% (after 4, 10 and 17 days, respectively) decrease in the AC positive type II collagen staining compared to that of the MC.

Aggrecan is a specific cartilaginous proteoglycan rich in sulfated glycosaminoglycans and serves as one of the major components of hyaline cartilage. Immunohistochemical analysis of 4 and 17 day old cultures (figure 5A), reveal markedly higher positive aggrecan activity in MC chondrocytes as compared to the AC-derived chondrocytes.

### Higher proteoglycan sulphation in mature MC culture compared to AC culture

Maturation and senescence of cartilage is typified by a decrease in matrix production reflected in decreasing rates of proteoglycan sulphation. Newly produced aggrecan may be determined by following incorporation of radiolabeled sulphate into CPC (cetyl pyridinium chloride) insoluble proteoglycan fraction. To study the effects of prolonged culture period on the sulphation rate, cultures were labeled with radiolabeled sulphate for 48 hrs intervals, between 7-9 and 12-14 days of culture. Incorporation of sulphate into proteoglycans was assayed as described above. Results shown in figure 5C show a 37% decrease in sulphate uptake by MC between 9 and 14 days. These results also clearly show that at each time point the sulphate uptake by AC is markedly lower (by 81 % and 89% respectively) than those of MC culture.

In addition to the expression of cartilaginous specific genes, normally differentiating hyaline cartilage is also characterized by its concomitantly decreasing production of type I collagen. The expression of type I collagen was assayed immunohistochemically. Densitometry of IHC staining (Figure 6A), show that while the expression of type I collagen is gradually reduced in the MC-derived chondrocytes, the expression of type I collagen by AC cells gradually increases and reaches 79%, 146% and more than 140% higher levels than those of MC-derived cell, after 4, 10 and 17 days respectively indicating formation of fibrocartilage.

Type I collagen is associated with fibrocartilage, which is relatively inferior in terms of strength and durability compared to hyaline cartilage.

### Example 2: Cartilage film (membrane)

Porcine mandibular condyle-derived chondrocytes from the second passage, were plated on cover glasses at a concentration of 5 × 10⁵ cells/ml in 35 mm culture wells and cultured under same conditions as described above for MCDC. Cells were re-fed every 48 hr. At 12 days post-plating, an intact cartilage film developed. The cartilage film was rigid enough to be transferred and re-plated in a new culture dish. Throughout the first 24 hr, cartilage film was cultured with only 1 ml of medium to let it adhere to the plate. Further culturing continued under ordinary conditions. Development of the cartilage culture originating from the original cartilage film was followed morphologically and biochemically.

### Cartilaginous film formed by MC-derived chondrocytes

Mandibular condyle-derived chondrocytes rapidly differentiate in culture into cartilage forming cells, which secrete increasing amounts of type II collagen and aggrecan. Four days post cells plating, using conditions as described above, an initial cartilaginous film is formed. This cartilage tissue gradually thickens and by about the twelfth day becomes a solid membrane, which can be mechanically manipulated while preserving its original features to proliferate and re-differentiate. The cartilaginous film was replated in culture medium to test its ability to proliferate following transfer. A cartilaginous film that undergoes hyperproliferation upon transfer to new growth medium would be highly undesirable for cartilage repair.

The mechanical strength and elasticity of eth cartilage film is tested using methods and devices known in the art.

Figure 7 presents photos taken 4, 8 and 14 days post re-plating of the formed 12-day old cartilage film. Four days post re-plating (figure 7A) new cells (NC) began to sprout out from the 'old' cartilage film (CF). After 8 days these new chondrocytes began to develop polygonal shapes (figure 7B). Two weeks post re-plating, a new typical chondrocyte culture developed (figure 7C) occupying most of the new culture well. However the new culture did not exhibit cellular over-growth, and is clearly restrained by a border of cells (figure 7D, arrow), indicating that the secondary new culture (NC) preserves features of a primary culture and does not exhibit hyperproliferation.

Cartilage film preserves its capacity to undergo neo-proliferation upon replating.

One of the most crucial prerequisites for a successful, lesion repairing transplantation, is the ability of the cells' propagation and differentiation *in situ.* To study whether MC-derived cartilage film exhibits this feature, proliferation in 12 day old MC cartilage film that had been pilled off its substratum and replated in a new culture well was determined. Results depicted in figure 8 show that thymidine incorporation assessed 48 hrs after replating is 400% higher than that of a parallel cartilage film left in its original culture well. Arrows show proliferating cells.

Therefore, the cartilage film exhibits the essential features for cartilage repair: the ability to proliferate and form hyaline cartilage *in situ.*

### Example 3: Xenotransplantation of MCDC cells

Adjuvant induced rheumatoid arthritis (AIA) is an accepted experimental model for rheumatoid-induced articular damages. AIA rats undergo an acute disease phase lasting about 30 days and characterized by severe edema in the joints in general and in the knee joint in particular. When edema disappears, most rats still drag their hind legs due to the severe damage of the articular cartilage.

Seven AIA female Lewis rats were left to recover for 30 days post the acute AIA phase. Four sets of two AIA rats each were included in each treatment. Two million MCDC cells derived from rat (1), mouse (2) or porcine (3) were injected into the affected knee joints in 0.5 ml of PBS. Each cellular source was injected to both hind knees of two rats. One AIA rat was left untreated as a positive control (C+). One of its knees was injected with PBS alone. Morphological results were compared to those of an intact-naive rat as a negative control (C-).

Rats were kept separately to recover from transplantation. About three-four weeks after transplantation most of the treated rats moved more freely compared to the positive control. After 60 days, rats were sacrificed and knee joints were excised and processed for histological analysis.

All four joints of the same MCDC treatment or 2 joints of the controls were examined histologically. Representative histological results are shown in figure 8. In the naive rat, the joint was covered by a multi-cellular layer of hyaline cartilage. The articular cartilages of the non-treated AIA joints- both PBS injected and the untouched, were very thin with severe lesions and damage to the subchondral bone. In all implanted knees-using rat, mouse or porcine-derived MCDC cells, a new multilayer hyaline cartilage occupied by young chondrocytes surfaced the articular cartilage and showed similar morphology to that of the naïve joints.

The new cartilage cells were organized in a characteristic developmental cell gradient forming the typical anatomical shape of the articular cartilage, and more significant was the observation that no cellular overgrowth occurred. Figure 9 further shows that no infiltration of WBC took place. The lack of any rejection signs is also reflected in the viable morphology of the cells. Figure 9A shows the articular cartilage of a control animal (C-); figure 9B shows a cartilage cross section from a positive control animal in which AIA was induced; allogenic transplantation of rat MCDC cells transplanted into rat (9C), xenogeneic transplantation of mouse MCDC cells transplanted into rat (9D) and xenogeneic transplantation of porcine MCDC cells transplanted into rat (9E). Example 4: Implantation of chondrocyte film into goats

Goats were operated on their right leg generating two 10mm diameter by 2 mm deep lesions, one in the medial femoral condyle and the second in the lateral femoral condyle. The medial lesion was covered by a chondrocyte film prepared from porcine MCDC, and sutured to the lesion margin; while the lateral lesion was left untreated. Through a small opening left in the proximal region of the lesion, a 12 day old cartilage film 25 mm diameter by 0.1 mm wide was implanted into the formed lesion; the surgical opening was closed. Following 10 days of indoor housing, the goats returned to the farm and were given unrestricted activity.

### Results:

### Implant evaluation

Three months post chondrocytes implantation onto articular lesions, newly developed tissues are evaluated based on the gross morphology, histological and histochemical analyses, biochemical features and biomechanical stability.

### Gross morphology

Upon euthanasia, the articular surface of the knee joint will be examined by 3 blinded observers and scored for surface smoothness, integration of the lesion-fill edge and transparency of the filled tissue.

For the purpose of detecting signs of immunological reactions, synovial membrane and joint capsule were observed and photographed for morphological changes and particularly any inflammation signs. The nearest lymphatic node will be examined and processed for histological analyses. Synovial fluid was collected for analyzing cells population and immunoglobulin proteins content. Parallel analyses are conducted on the intact /counterpart leg.

### Tissue biopsies

Three osteochondral necropsies were taken, each a cylinder of 10mm diameter (4 mm larger than the original lesion) to include the lesion-fill/adjacent tissue interface, 5 mm depth (including ∼2 mm of the subchondral bone).
a. Explant of the treated (cells implantation) lesion on the medial femoral condyle.
b. Explant of the non-treated lesion on the lateral femoral condyle.
c. Explant of an intact articular cartilage from the medial condyle.

### Gross analysis of goat's knee joints post cartilage film implantation

Fourteen weeks following cartilage film implantation into the medial femoral articular lesion, one goat was sacrificed, synovial fluid was drained and analyzed and gross morphology of the lesions was documented. The three month interval was chosen for following the healing process, since no complete lesion repair is expected before 6 months. Results show that both lesions were not yet totally covered. However, the dimensions of the treated lesion was 20% smaller while those of the untreated lesion were 20% higher than the original lesion size. The untreated lesion became irregularly shaped as opposed to the regular rounded shape of the original and of the treated lesion.

Synovial fluid composition was similar in the operated (right) and intact (left) joint. Although the operated joint contained more fluid, the total neutrophil cell number was identical. Goats move freely, eat and behave normally. All blood test results were within normal ranges; including white blood cell (WBC) count. Joint gross morphology was unchanged with no signs of edema. Synovial fluid drained 3 months after implantation had no signs of inflammation.

Results shown in table 1 clearly show that the increased SF volume in the treated joint is not due to inflammatory reaction, but rather to normal healing process often followed by increased secretion of SF. The total neutrophil number, indicating a potential inflammatory state, is similar in both joints; (in the left joint the neutrophil concentration is 4 times higher, in a 4 times smaller volume). Table 1: Synovial Fluid

| **Joint** | **Total fluid** | **WBC** | **Neutrophils** |
|---|---|---|---|
| **Right leg (operated)** | 1000 µl | 0.2 x 10³ _{.}/µl | 4% |
| **Left (intact)** | 250 µl | 0.24 x 10³ _{.}/µl | 19.6% |

Gross morphology of the lesions: Both lesions were measured and photographed before being subjected to histological and biochemical analysis.

Lesion dimensions are shown in table 2. The untreated lesion size (on the lateral condyle) increased by 21% while the treated lesion size (on the medial condyle) decreased by 20%.

**Table 2: Changes in lesions size after 3 months.**

| **Joint** | **Original lesion size** | **Lesion size after 14 weeks** |
|---|---|---|
| **Right** | 314 mm² | 254 mm² |
| **Left** | 314 mm² | 380 mm² |

Photographs of the lesions are presented in Figure 10. The treated-medial lesion is rounded and shows signs of initial cartilage covering at the lesion's margins (Figure 10C).

The lateral lesion (untreated) has developed an irregular shape, indicating a possible preliminary secondary osteoarthritic process, a well known phenomenon that often accompanies untreated articular lesions (Figure 10B).

### Tracing the implanted cells

Tracing the localization of the porcine derived implanted chondrocytes will be performed based on gender differences: the implanted chondrocytes are derived from male porcine and the recipient is female goat. A Y-chromosome specific gene will serve for localizing the porcine-derived cells within the recovered articular lesion and to trace any porcine originated cells that might leak out of the implant into the synovial fluid. For this purpose the expression of the porcine Y gene will be localized in the synovial fluid and in osteochondral biopsies' sections of the using PCR and in situ hybridization respectively.

### Biomechanical stability studies

Biomechanical evaluations of the newly formed cartilage, necropsies of osteochondral (articular cartilage and its subchondral bone) harvested from the treated lesions, the untreated lesions and from parallel healthy tissues in the counter (intact) knee, will be made immediate post tissue excising. Specimens are immersed in Phosphate-Buffered Saline (PBS, pH 7.2) containing protease inhibitors (1 mM phenylmethanesulfonyl fluoride, 2 mM disodium ethylenediamine tetraacetate, 5 mM benzamidine-HCl, and 10 mM N-ethylmaleimide). Tissues are assayed within 30 min of their harvesting.

Rigidity of the tissue is assessed by indentation test using 'universal testing apparatus 4502'-Instron-Bioplus™, and a 0.3mm plane ended indenter. The loading protocol consists of application of a "tare displacement" (for 30 s) and then four "test displacements" of 3 s duration each. For each tare displacement-load at 0 s and 3 s is noted. Tare displacement is 25 micrometer, test displacements are 50,100, 200, and 300 micrometers. Indentation Stiffness is computerized calculated as the slope of load-displacement data, in units of N/mm (with no need to regard indenter diameter).

### Histological and biochemical tests

Immediately after the biomechanical analyses, explants will be cut at a perpendicular axis to the lesion, through the center: one section will be used for histological/histochemical studies and the other for biochemical analyses.

### Histochemical/immunohistological analyses

Explants are fixed with NBF (neutral buffered formalin), decalcified for 48 hr in EDTA, dehydrated in gradual alcohols and routinely processed for paraffin embedding.

Six millimeter (mm) thick sections perpendicular to the lesion plane are mounted on pre-cleaned poly-lysine coated slides and are used for histochemical and immunohistological staining.

### Histochemical staining

For general histological analysis sections are stained with Hematoxilin / Eosin, and with acidic Alcian blue and/or Safranin O for detection of cartilage proteoglycan. To exclude ossification of the growing tissue, von Kossa staining for calcified tissue is performed.

### Immunohistochemical analyses

To further characterize the nature of the developing cartilage, the following antigens are localized using immunohistochemistry and relevant antibodies:
· type I, II and X collagen for detection of fibrocartilage, hyaline cartilage and hypertrophic cartilage respectively (for collagen determination, sections are pre-treated for 7 min with 0.1 % pepsin) ,
· BMP2 and FGFR3: two cartilage-specific proteins involved in paracrine regulatory activity,
· Aggrecan and Osteocalcin: cartilage and bone specific non-collagenous proteins respectively.

### Biochemical analyses

The second explant samples will serve for extraction of lysate proteins, DNA and RNA for immunoblotting, PCR and real time PCR analysis respectively.

### Immunoblotting

For quantification of cartilage specific proteins, spectrophotometry of immunoblotting analyses will be performed using the antibodies listed above for immunohistochemistry.

### Real time PCR (RT-PCR)

Quantitative analysis of the expression (mRNA level) of the cartilage specific genes, will be determined using RT-PCR and specific primers for type I and II collagen and aggrecan. Programming of the specific primers will be made with the assistance of the RT-PCR manufacturer company support.

PCR analysis of porcine Y gene:
PCR analysis of the synovial cells DNA is performed using oligonucleotide primers SEQ ID NO:5 and SEQ ID NO:6 (see table 3) for the 236 bp fragment of porcine male-specific DNA sequence and 1.25 104 template white blood cells obtained from a boar.

In situ hybridization (ISH) and probe labeling:
In situ hybridization (ISH) is performed on deparaffinized sections using digoxigenin (Dig)-labeled antisense mRNA of the following probes: core protein of aggrecan, osteocalcin and collagens: types II and X. Dig-labeled probes are produced by two PCR steps. The first product will be produced using primers detailed below. The first PCR product is subjected to PCR amplification using promoter-primer sequence combined to the gene specific primer as brought in the table. The second product serves as a template for either sense (negative control) or antisense (in situ hybridization) Dig-labeled RNA using (Sp6/T7) Dig-RNA labeling kit (Roche, Germany), following the manufacturer's instructions. Hybridization conditions and detection will be performed as described below.

For localizing Y chromosome gene, a Dig labeled DNA probe will be performed, using PCR Dig probe synthesis kit (Roche).

Table 3 provides exemplary primer sequences for use in, *inter alia,* the RT-PCR and ISH procedures.

**Table 3**

| | Primer sequence | size |
|---|---|---|
| Collagen type II - sense | T7 promoter + TCTCCTGCCTCCT (SEQ ID NO :1 ) | 302bp |
| Collagen type II - antisense | T3 promoter + CCATCTCTGCCACGG (SEQ ID NO :2) | |
| Aggrecan forward | T7 promoter + GTCCTCTCCAGT (SEQ ID NO :3) | 345bp |
| Aggrecan reverse | T3 promoter + TTGCTTCTCCAGA (SEQ ID NO :4) | |
| Porcine Y-chromosome -sense | T7+AAGTGGTCAGCGTGTCCATA (SEQ ID NO :5) | 236 bp |
| Porcine Y-chromosome antisense | T3 + TTTCTCCTGTATCCTCCTGC (SEQ ID NO :6) | |
| Collagen type X - antisense | T7 promoter + GATCCTCACATA (SEQ ID NO :7) | 481bp |
| Collagen type X - sense | T3 promoter + ACCTGTAAGATCC (SEQ ID NO :8) | |
| Osteocalcin - antisense | T7 promoter + TCATCTGAACTTTA (SEQ ID NO :9) | 437bp |
| Osteocalcin - sense | T3 promoter + ACACCTAGCAGACAC (SEQ ID NO :10) | |

The T7 promoter primer sequence is
5' CAAGCTTCTAATACGACTCACTATAGGGAGA 3' (SEQ ID NO:11);

The T3 promoter primer sequence is
5' CCAAGCTTCATTAACCCTCACTAAAGGGAGA 3'(SEQ ID NO:12).

### In situ hybridization (ISH)

In situ hybridization is performed on deparaffinized sections loaded on precleaned poly-L-lysine-coated slides. Sections are treated with 3% H2O2 in methanol to neutralize endogenous peroxidase. After permeabilization with 12.5 mg/ml proteinase K for 15 min, (stopped with 2 mg/ml glycine), and acetylation in 0.5% acetic anhydride in 0.1 mol/L Tris at pH 8.0, sections are post-fixed with 4% paraformaldehyde/phosphate buffered saline (PBS). Prehybridization for 10 min in 2 x standard saline citrate (2xSSC) is followed by one hour in hybridization buffer: 50% formamide, 0.5 mg/mL salmon sperm DNA, 4xSSC, 1x Denhardt's, 200 U/ml heparin, 5% dextran sulfate and 0.01% sodium dodecyl sulfate (SDS). Hybridization is carried out for 18hr at 42°C and maximal humidity with a 5mg/mL digoxygenin labeled antisense RNA probe (see above). Digoxygenin labeled sense RNA probes were run as negative controls. At the end of the incubation period, slides are rinsed in SSC under increasingly stringent conditions and then with 0.1mol Tris, 0.15 mol/L NaCl at pH 7.5. Hybrids are detected using anti-digoxygenin antibodies conjugated with peroxidase (Boehringer Mannheim, Germany) and AEC as a substrate; counterstaining is done with hematoxylin. For ISH of positive porcine Y gene, slides will undergo similar pre-treatment except for acetylation. Prior to hybridization, slides- covered with the labeled probe cocktail, are prewarmed for 6 min at 95°C, cool immediately and placed to hybridize for 3 hrs at 42°C.

### Conclusions

Mandibular condyle derived chondrocytes (MCDC/MC) provide a unique source of cartilage producing cells different from other cartilage sources, in particular from articular cartilage (AC). AC is the standard source for chondrocytes used in replacement procedures and the only one currently approved for autologous cartilage transplantation (ACT).

Comparison between these two sources of chondrocytes clearly shows that MC is preferable to AC as a source for cartilage producing cells for the purpose of treating articular lesions from both efficacy and competency aspects. Viable cell population yielded from MC is much higher than that of AC. The cells are more metabolically active and proliferate at a higher rate than AC chondrocytes. Most important, MC derived chondrocytes express higher levels of type II collagen and aggrecans than AC cells, indicating spontaneous differentiation of MC cells into hyaline cartilage producing cells. AC cultures, in contrast, produce decreasing amounts of type II collagen and aggrecan with culturing time, and express increasing amounts of type I collagen, indicating development of potentially detrimental fibrocartilage.

Prolonged MCDC cultures develop into cartilage film composed of chondrocytes and extracellular matrix containing mainly type II collagen and aggrecans. After 10-14 days of culture the cartilage film is rigid enough to be mechanically manipulated yet pliable enough to adapt to various contours of particular cartilage's shape. It can be transferred from one dish to another as an intact tissue (replating). Upon transfer, the re-cultured film regains developmental activities featuring the newly cultured MCDC chondrocytes, indicating that the cartilage film preserves *de novo* chondrogenesis potential including cell proliferation and differentiation. At the margins of the film, cells start to proliferate, followed by differentiation into hyaline cartilage. The growth of the cartilage is restrained, and no overgrowth of cartilage tissue is observed. These unique features of the cartilage film provide a product suitable for cartilage replacement since it guarantees *in situ* development of vital, high quality hyaline cartilage.

The therapeutic potential of xenograft MCDC cells was initially shown using a rat model for rheumatoid arthritis (RA). RA is considered an autoimmune disease causing severe damage to the joints' articular cartilage. Utilizing an Adjuvant Induced rheumatoid Arthritis (AIA), an experimental model for RA, we could show that either allogenic (rat) derived MCDC chondrocytes, or xenogenic (mouse or porcine) derived cells replenished the articular lesions caused by AIA. Newly formed cartilage displayed typical developmental cell gradient restrained to the anatomical boundaries of the apical epiphysis. Moreover, no signs of any immunological response were noticed and WBC were not observed. These results support the advantages of using xenografts MCDC chondrocytes for articular lesion replacement without provoking any undesired immunological response

While the present invention has been particularly described, persons skilled in the art will appreciate that many variations and modifications can be made. Therefore, the invention is not to be construed as restricted to the particularly described embodiments, rather the scope, spirit and concept of the invention will be more readily understood by reference to the claims which follow.

### REFERENCES

Atagunduz P, Appel H, Kuon W, Wu P, Thiel A, Kloetzel PM, Sieper J: Hla-B27-Restricted Cd8+ T Cell Response To Cartilage-Derived Self Peptides In Ankylosing Spondylitis. Arthritis Rheum 52:892-901, 2005
Briggs TW, Mahroof S, David LA, Flannelly J, Pringle J, Bayliss M. Histological evaluation of chondral defects after autologous chondrocyte implantation of the knee. Journal of Bone & Joint Surgery. British Volume. 85(7):1077-83, 2003
Chu CR, Convery FR, Akeson WH, Meyers M, Amiel D: Articular Cartilage Transplantation. Clinical Results In The Knee. Clin Orthop Relat Res:159-168, 1999
Elves MW: Newer Knowledge Of The Immunology Of Bone And Cartilage. Clin Orthop Relat Res:232-259, 1976
Fuentes-Boquete I, Lopez-Armada MJ, Maneiro E, Fernandez-Sueiro JL, Carames B, Galdo F, de Toro FJ, Blanco FJ. Pig chondrocyte xenoimplants for human chondral defect repair: an in vitro model. Wound Repair Regen. 12(4):444-52, 2004
Hunziker, EB. Articular cartilage repair: basic science and clinical progress. A review of the current status and prospects. Osteoart. Cart., 10:432-465, 2002
Hutmacher DW, Ng KW, Kaps C, Sittinger M, Klaring S: Elastic Cartilage Engineering Using Novel Scaffold Architectures In Combination With A Biomimetic Cell Carrier. Biomaterials 24:4445-4458, 2003
Kandel RA, Gross AE, Ganel A, McDermott AG, Langer F, Pritzker KP: Histopathology Of Failed Osteoarticular Shell Allografts. Clin Orthop Relat Res: 103-110, 1985
Lu Y, Adkisson HD, Bogdanske J, Kalscheur V, Maloney W, Cheung R, Grodzinsky AJ, Hruska KA, Markel MD: In Vivo Transplantation Of Neonatal Ovine Neocartilage Allografts: Determining The Effectiveness Of Tissue Transglutaminase. J Knee Surg 18:31-42, 2005
Mahomed MN, Beaver RJ, Gross AE: The Long-Term Success Of Fresh, Small Fragment Osteochondral Allografts Used For Intraarticular Post-Traumatic Defects In The Knee Joint. Orthopedics 15:1191-1199, 1992
Marco F, Lopez-Oliva F, Fernandez Fernandez-Arroyo JM, De Pedro JA, Perez AJ, Leon C, Lopez-Duran L: Osteochondral Allografts For Osteochondritis Dissecans And Osteonecrosis Of The Femoral Condyles. Int Orthop 17:104-108, 1993
Martinek V, Usas A, Pelinkovic D, Robbins P, Fu FH, Huard J: Genetic Engineering Of Meniscal Allografts. Tissue Eng 8:107-117, 2002
McDermott AG, Langer F, Pritzker KP, Gross AE: Fresh Small-Fragment Osteochondral Allografts. Long-Term Follow-Up Study On First 100 Cases. Clin Orthop Relat Res:96-102, 1985
Meyers MH, Akeson W, Convery FR: Resurfacing Of The Knee With Fresh Osteochondral Allograft. J Bone Joint Surg Am 71:704-713, 1989
Moskalewski S, Hyc A, Osiecka-Iwan A: Immune Response By Host After Allogeneic Chondrocyte Transplant To The Cartilage. Microsc Res Tech 58:3-13, 2002
Pollok JM, Kolln PA, Lorenzen M, Torok E, Kaufmann PM, Kluth D, Bohuslavizki KH, Gundlach M, Rogiers X: Islets Of Langerhans Encapsulated With A Tissue-Engineered Membrane Of Rat Chondrocytes Maintain Insulin Secretion And Glucose-Insulin Feedback For At Least 30 Days In Culture. Transplant Proc 33:1713-1714,2001
Pollok JM, Lorenzen M, Kolln PA, Torok E, Kaufmann PM, Kluth D, Bohuslavizki KH, Gundlach M, Rogiers X: In Vitro Function Of Islets Of Langerhans Encapsulated With A Membrane Of Porcine Chondrocytes For Immunoisolation. Dig Surg 18:204-210, 2001

Van Bilsen JH, Van Dongen H, Lard LR, Van Der Voort EI, Elferink DG, Bakker AM, Miltenburg AM, Huizinga TW, de Vries RR, Toes RE: Functional Regulatory Immune Responses Against Human Cartilage Glycoprotein-39 In Health vs. Proinflammatory Responses In Rheumatoid Arthritis. Proc Natl Acad Sci U S A 101:17180-17185, 2004

## Claims

1. Use of a flexible chondrocyte film prepared from a mammalian perinatal mandibular condyle derived chondrocytes (MCDC) for the treatment of a disorder selected from a cartilage lesion and a subchondral bone lesion in a subject.

2. A pharmaceutical composition comprising a flexible chondrocyte film prepared from a mammalian perinatal mandibular condyle derived chondrocytes (MCDC).

3. A pharmaceutical composition comprising a flexible chondrocyte film prepared from a mammalian perinatal mandibular condyle derived chondrocytes (MCDC) associated with a biocompatible scaffold.

4. The use of claim 1, wherein said cartilage lesion results from a disease or injury, preferably wherein said disease is a cartilage degenerative disease selected from osteoarthritis or alternatively preferably wherein the injury is a sports injury.

5. A method for preparing the flexible film of claim 1, the method comprising:
(a) isolating MCDC from the mandibular condyle of a perinatal mammal;
(b) culturing the chondrocytes under conditions to induce attachment of the chondrocytes to a surface and secretion of type II collagen matrix;
(c) culturing the chondrocytes from one to about 10 cell layers thick; and
(d) separating the film from the surface, as a film.

6. The use of claim 1, pharmaceutical composition of claim 2 or 3 or method of claim 5, wherein the film is 35 mm diameter by 0.1 mm wide.

7. The use of claim 1 or pharmaceutical composition of claim 2 or 3, wherein said chondrocytes are surrounded by an insoluble matrix which comprises type II collagen.

8. The use of claim 1 or pharmaceutical composition of claim 2 or 3, wherein said film comprises about 2 to about 5 layers of MCDC.

9. The use of claim 1 or pharmaceutical composition of claim 2 or 3, wherein said film further comprises aggrecan.

10. The use of claim 1 or pharmaceutical composition of claim 2 or 3, wherein said MCDC are isolated from a preinatal donor mammal selected from the group consisting of a neonate and a fetus.

11. The use of claim 10 pharmaceutical composition of claim 2 or 3, wherein said neonate is less than 7 days old, preferably less than 3 days old.

12. The use of claim 1, wherein said chondrocyte film does not elicit an immune response that is detrimental to the retention of the graft.

13. The use of claim 1 or method of claim 5, wherein said MCDC are allogeneic or xenogeneic to the subject.

14. The use or method of claim 13, wherein said xenogeneic MCDC are derived from a pig.

15. The use of claim 1, wherein said film is associated with a biocompatible scaffold.


SEQUENCE LISTING

<110>  CARTICURE LTD.
       MAOR, Gila

<120>  METHOD FOR NON-AUTOLOGOUS CARTILAGE REGENERATION

<130>  NGT/001 PCT

<140>  PCT/IL2006/
<141>  2006-11-12

<150>  US 60/813,690
<151>  2006-06-15

<150>  US 60/735,204
<151>  2005-11-10

<160>  12

<170>  PatentIn version 3.3

<210>  1
<211>  13
<212>  DNA
<213>  ARTIFICIAL

<220>
<223>  primer

<400>  1
tctcctgcct cct                                                          13


<210>  2
<211>  15
<212>  DNA
<213>  ARTIFICIAL

<220>
<223>  primer

<400>  2
ccatctctgc cacgg                                                        15


<210>  3
<211>  12
<212>  DNA
<213>  ARTIFICIAL

<220>
<223>  primer

<400>  3
gtcctctcca gt                                                           12


<210>  4
<211>  13
<212>  DNA
<213>  ARTIFICIAL

<220>
<223>  primer

<400>  4
ttgcttctcc aga                                                          13


<210>  5
<211>  20
<212>  DNA
<213>  ARTIFICIAL

<220>
<223>  primer

<400>  5
aagtggtcag cgtgtccata                                                   20


<210>  6
<211>  20
<212>  DNA
<213>  ARTIFICIAL

<220>
<223>  primer

<400>  6
tttctcctgt atcctcctgc                                                   20


<210>  7
<211>  12
<212>  DNA
<213>  ARTIFICIAL

<220>
<223>  primer

<400>  7
gatcctcaca ta                                                           12


<210>  8
<211>  13
<212>  DNA
<213>  ARTIFICIAL

<220>
<223>  primer

<400>  8
acctgtaaga tcc                                                          13


<210>  9
<211>  14
<212>  DNA
<213>  ARTIFICIAL

<220>
<223>  primer

<400>  9
tcatctgaac ttta                                                          14


<210>  10
<211>  15
<212>  DNA
<213>  ARTIFICIAL

<220>
<223>  primer

<400>  10
acacctagca gacac                                                         15


<210>  11
<211>  31
<212>  DNA
<213>  ARTIFICIAL

<220>
<223>  primer (T7)

<400>  11
caagcttcta atacgactca ctatagggag a                                       31


<210>  12
<211>  31
<212>  DNA
<213>  ARTIFICIAL

<220>
<223>  primer (T3)

<400>  12
ccaagcttca ttaaccctca ctaaagggag a                                       31